# EUROPEAN PATENT SPECIFICATION

(11) **EP 1 286 665 B1**
(45) Date of publication and mention of the grant of the patent: **17.04.2013**
(21) Application number: 01935167.5
(22) Date of filing: 09.05.2001
(51) Int. Cl.: A61K 31/662, A61K 9/08, A61P 19/08, A61P 35/00, A61K 9/19

(54) **TREATMENT REGIMEN OF INTRAVENOUS ZOLEDRONATE FOR THE TREATMENT OF BONE METABOLISM DISEASES**
BEHANDLUNGSSCHEMA VON ZOLEDRONAT ZUR BEHANDLUNG VON ERKRANKUNGEN DES KNOCHENMETABOLISMUS
SCHEMA DE TRAITEMENT DU ZOLEDRONATE POUR LES MALADIES DE METABOLISME OSSEUX

(30) Priority: 19.05.2000 GB 0012209
(43) Date of publication of application: 05.03.2003
(73) Proprietor: Novartis AG, 4056 Basel (CH); Novartis Pharma GmbH, 1230 Wien (AT)
(72) Inventor: SEAMAN, John, J., New Hope, PA 18938 (US); GALLI, Bruno, 4411 Seltisberg (CH); SCHRAN, Horst, Morristown, NJ 07960 (US)
(74) Representative: Trösch, Dominique
(86) International application number: PCT/US2001/014886
(87) International publication number: WO 2001/089494

(56) References cited:
- BERENSON J R ET AL: "A phase I dose-ranging trial of monthly infusions of zoledronic acid for the treatment of osteolytic bone metastases." CLINICAL CANCER RESEARCH, (2001 MAR) 7 (3) 478-85. , XP001036868
- BERENSON JAMES R ET AL: "Zoledronic acid reduces skeletal-related events in patients with osteolytic metastases: A double-blind, randomized dose-response study." CANCER, vol. 91, no. 7, 1 April 2001 (2001-04-01), pages 1191-1200, XP001036871 ISSN: 0008-543X
- BODY J J: "Clinical research update: Zoledronate." CANCER, vol. 80, no. 8 SUPPL., 1997, pages 1699-1701, XP001037214 ISSN: 0008-543X
- LIPTON A ET AL: "Phase II trial of zoledronate vs. pamidronate in multiple myeloma and breast cancer." CANCER INVESTIGATION., vol. 18, no. SUPPL. 1, 2000, pages 68-69, XP001036876 XVII Symposium of the Chemotherapy Foundation: Innovative Cancer Therapy for Tomorrow.;New York, NY, USA; November 03-06, 1999 ISSN: 0735-7907
- BUCKLER H ET AL: "Single infusion of zoledronate in Paget's disease of bone: A placebo-controlled, dose-ranging study." BONE (NEW YORK), vol. 24, no. 5 SUPPL., May 1999 (1999-05), pages 81S-85S, XP001036896 International Symposium of the 25th Anniversary of the National Association for the Relief of Paget's Disease;Oxford, England, UK; July 15-17, 1998 ISSN: 8756-3282
- Publication of Swiss marketing approval for Zometa® 4 mg, Lyophilisat in the IKS Monatsbericht 11/2000
- Summary of Product Characteristics for Zometa® by Novartis
- Product monograph for Zoledronate Disodium (Zometa) in Drugs of the Future, March 2000, 25(3), 259-268
- GREEN J R ET AL: "Renal tolerability profile of novel, potent bisphosphonates in two short-term rat models.", PHARMACOLOGY & TOXICOLOGY MAY 1997, vol. 80, no. 5, May 1997 (1997-05), pages 225-230, ISSN: 0901-9928
- KANIS J A ET AL: "Effects of intravenous etidronate disodium on skeletal and calcium metabolism.", THE AMERICAN JOURNAL OF MEDICINE 23 FEB 1987, vol. 82, no. 2A, 23 February 1987 (1987-02-23), pages 55-70, ISSN: 0002-9343
- MIAN M ET AL: "Tolerability and safety of clodronate therapy in bone diseases.", INTERNATIONAL JOURNAL OF CLINICAL PHARMACOLOGY RESEARCH 1991, vol. 11, no. 2, 1991, pages 107-114, ISSN: 0251-1649
- 'Definition of Infusion', [Online] page 1 Oxford Dictionaries Retrieved from the Internet: <URL:http://oxforddictionaries.com/definiti on/english/infusion?q=infusion> [retrieved on 2012-09-06]
- 'Route of Administration', [Online] pages 1 - 6 Wikipedia Retrieved from the Internet: <URL:http://en.wikipedia.org/wiki/Route_of_ administration> [retrieved on 2012-09-06]
- MAJOR P ET AL: "Zoledronic acid is superior to pamidronate in the treatment of hypercalcemia of malignancy: a pooled analysis of two randomized, controlled clinical trials.", JOURNAL OF CLINICAL ONCOLOGY : OFFICIAL JOURNAL OF THE AMERICAN SOCIETY OF CLINICAL ONCOLOGY 15 JAN 2001, vol. 19, no. 2, 15 January 2001 (2001-01-15), pages 558-567, ISSN: 0732-183X
- NAKASHIMA L.: "Guidelines for the treatment of hypercalcemiaassociated with malignancy", J. ONCOL. PHARM. PRACTICE, vol. 3, no. 1, 1997, pages 31-37,
- Affidavit of Dr. Horst Schran filed with the USPTO in 24.04.2006 during the prosecution of the equivalent US patent application US2004/0014724-A1
- Berenson et al; Population pharmacokinetics (PK) of Zometa, 2000, Proceedings of ASCO, Vol. 19, Clinical Pharmacology, 209a, No. 814
- BERENSON J R: "Zoledronic acid in cancer patients with bone metastases: results of Phase I and II trials.", SEMINARS IN ONCOLOGY APR 2001, vol. 28, no. 2 Suppl 6, April 2001 (2001-04), pages 25-34, ISSN: 0093-7754
- Fleisch; Bisphosphonates in bone disease, 1997, third edition, p.152.
- DOCCHECK FLEXIKON: 'Parenteral', [Online] pages 1 - 1 Retrieved from the Internet: <URL:http://flexikon.doccheck.com/de/Parent eral> [retrieved on 2013-01-08]
- ROSEN LEE S ET AL: "Long-term efficacy and safety of zoledronic acid compared with pamidronate disodium in the treatment of skeletal complications in patients with advanced multiple myeloma or breast carcinoma: a randomized, double-blind, multicenter, comparative trial.", CANCER 15 OCT 2003, vol. 98, no. 8, 15 October 2003 (2003-10-15), pages 1735-1744, ISSN: 0008-543X

## Description

The present invention relates to a bisphosphonate, specifically 2-(imidazoh-1yl)-1-hydroxyethane-1,1-diphosphonic add (zoledronic acid) or pharmaceutically acceptable salts thereof of use in the treatment of bone metabolism disease according to claim 1.

Bisphosphonates are widely used to inhibit osteoclast activity in a variety of both benign and malignant diseases that involve excessive or inappropriate bone resorption. These pyrophosphate analogues not only reduce the occurrence of skeletal related events but they also provide patients with clinical benefit and improved survival. Bisphosphonates are able to prevent bone resorption *In vivo;* the therapeutic efficacy of bisphosphonates has been demonstrated in the treatment of osteoporosis, osteopenia, Paget's disease of bone, tumour-induced hypercalcemia (TIH) and, more recently, bone metastases (BM) and multiple myeloma (MM) (for review see Fleisch H 1997 Bisphosphonates clinical. In Bisphosphonates in Bone Disease, From the Laboratory to the Patient. Eds: The Parthenon Publishing Group, New York/London pp 68-163).

Recently, it has been reported that bisphosphonate (dodronate, etidronate, alendronate and pamidronate) administration has a favourable effect on bone pain in patients with metastatic prostate cancer carcinoma (Silvio Adami, Cancer 1997; 80: 1674-79). Recently, it has also been reported that bisphosphonates inhibit breast and prostate carcinoma cell adhesion to bone *in vitro* (Boissier et al., Cancer Res; 57: 3890-3894, 1997) and further that pre-treatment of breast and carcinoma cells with bisphosphonates Inhibited tumour cell invasion via a direct action on the tumour cells. Yet more recently, it has been reported that *in vitro* treatment of prostate cancer cell lines with zoledronic acid significantly reduced the growth of the cell lines (Brown et al. Effects of Zoledronate on Prostate Cancer Cells, ASBMR 2000; Lee et al., Bisphosphonate Treatment inhibits the Growth of Prostate Cancer Cells, Cancer Research, 2000/2001); whereas no significant decrease in tumour volume was detected for subcutaneous prostate cancer cell line tumours treated with zoledronic acid (Corey et al., Effects of Zoledronic Add on Prostate Cancer in Vitro and in Vivo, Amer. Assoc. Cancer Res. Submitted Oct 2000). The swiss summary of product characteristics for Zomela provides further information on the use of zoledronic acid.

Additionally, it has now been shown in a double blind, placebo-controlled clinical study that zoledronic acid (zoledronate) demonstrates a statistically significant efficacy benefit over placebo in the treatment of bone metastases In prostate cancer patients and that bisphosphonates may also be employed more generally for the treatment of osteoblastic (osteosclerotic) metastases, In particular osteoblastic bone metastases, such as the osteoblastic metastases associated with prostate cancer and similar malignant diseases In mammals.

1-hydroxy-2(1H-Imidazol-1-yl)-phosphono-ethyl phosphonic acid (zoledronic acid, zoledronate) is a third generation bisphosphonate compound. In animal models zoledronic acid shows high affinity to the mineralised bone matrix and inhibits osteoclastic bone resorption more effectively than earlier generation bisphosphonates, at doses that do not affect bone formation and mineralization and have no appreciable impact on renal function. This results in an improved ratio of antiresorptive versus renal effects (Green *et al.,* 1994; Green *et al*., 1997). Zoledronic acid (ZOMETA™)^{ls} currently under regulatory review for the treatment of tumor-induced hypercalcemia (TIH) on the basis of safety and efficacy data in a dose finding study (Vigneron *et al*., 1995) and two pivotal clinical trials (Mull *et al.,* 1999; O'Neill *et al.,* 1999), as well as supporting safety data from several other studies in cancer patients with bone metastases (van Valen et al., 1999; Goas *et al.,* 1999; Borg *et al*., 1999). The clinical studies demonstrate that the pharmacological action of zoledronic add in reducing osteoclastic hyperactivity results in an effective clinical inhibition of bone resorption and calcium release into blood in TIH patients.

It has been found that an intravenously administered 4mg dose of zoledronic add infused over an interval of approximately 15 minutes showed 1) improved clinical practicality, 2) potentially more reproducible infusion rate when using 100 ml over 15 min vs. lower volume infused over shorter period, 3) shows comparable efficacy to the current standard treatment, Aredia^{®} (disodium pamidronate) 90 mg dosed over a period of 2-4 hours, and 4) 4 mg/15 minutes shows improved renal safety versus 4 mg/ 5 minutes and higher zoledronic add dose/15 minutes. Thus in one embodiment, the invention is directed to 2-(imidazol-1yl)-1-hydroxyethane-1,1-diphosphonic acid (zoledronic acid, zoledronate) or pharmaceutically acceptable salts thereof for use in the treatment of bone metabolism disease, wherein 4mg of 2-(imidazol-1yl)-1-hydroxyethane-1,1-diphosphonic acid is to be administered intravenously over a period of 15 minutes in an infusion volume of 100 ml to a patient in need of said treatment.

In another embodiment, the present invention relates to 2-(imidazol-1yl)-1-hydroxyethane-1,1-diphosphonic acid or pharmaceutically acceptable salts thereof for use in the treatment of bone metabolism diseases, e.g., tumour induced hypercalcemia (TIH), prosthesis loosening, treatment or reversal pf angiogenesis associated with pathological conditions, e.g. tumour angiogenesis, wherein 4mg of 2-(imidazol-1yl)-1-hydroxyethane-1,1-diphosphonic acid is to be administered intravenously over a period of 15 minutes in an infusion volume of 100 ml to a patient in need of said treatment.

In a further embodiment, the present invention relates to 2-(imidazol-1yl)-1-hydroxyethane-1,1-diphosphonic acid or pharmaceutically acceptable salts thereof for use in the treatment of bone metastases, wherein 4mg of 2-(imidazol-1 yl)-1-hydroxyethane-1,1-diphosphonic acid is to be administered intravenously over a period of 15 minutes in an infusion volume of 100 ml to a patient in need of said treatment.

In a still further embodiment, the present invention relates to 2-(imidazol-1yl)-1-hydroxyethane-1,1-diphosphonic acid or pharmaceutically acceptable salts thereof for use in the treatment of multiple, myeloma, wherein 4mg of 2-omidazol-1yl)-1-hydroxyethane-1,1-diphosphonic acid is to be administered intravenously over a period of 15 minutes in an infusion volume of 100 ml to a patient in need of said treatment.

In yet another embodiment the present invention is a pharmaceutical composition containing 4 mg of therapeutically effective 2-(imidazol-1yl)-1-hydroxyethane-1,1-diphosphonic acid for use in the treatment of bone metabolism disease, wherein 4 mg of 2-(imidazol-1-yl)-1-hydroxyethane-1,1-diphosphonic acid is to be administered intravenously over a period of 15 minutes in an infusion volume of 100 ml to a patient in need in need of said treatment.

In a further embodiment the present invention is a pharmaceutical composition containing 4 mg of therapeutically effective 2-(imidazol-1yl)-1-hydroxyethane-1,1-diphosphonic acid for use in the treatment of bone metastases of multiple myeloma, wherein 4 mg of 2-(imidazol-1yl)-1-hydroxyethane-1,1-diphosphonic acid is to be administered intravenously over a period of 15 minutes in an infusion volume of 100 ml to a patient in need of said treatment.

In the present description the terms "treatment" or "treat" refer to both prophylactic or preventative treatment as well as curative or disease modifying treatment, including treatment of patients at risk of developing TIH, BM, or MM or suspected to have contracted the disease, e.g. TIH, as well as patients who are III or have been diagnosed as suffering from a particular disease or medical condition treatable with bisphosphonates.

Zoledronic acid, chemically designated as 1-hydroxy-2-(Imidazol-1-yl)ethane-1,1-diphosphonic acid having the structure 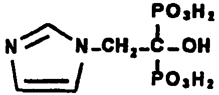 is known and can be prepared as described e.g. in US patent 4,939,130 (see also US patents 4,777,163 and 4,687,767).

Pharmaceutically acceptable salts of 1-hydroxy-2-(Imidazol-1-yl)ethane-1,1-diphosphonic acid are preferably salts with bases, conveniently metal salts derived from groups la, Ib, IIa and IIb of the Periodic Table of the Elements, including alkali metal salts, e.g. potassium and especially sodium salts, or alkaline earth metal salts, preferably calcium or magnesium salts, and also ammonium salts with ammonia or organic amines.

Especially preferred pharmaceutically acceptable salts are those where one, two, three or four, in particular one or two, of the acidic hydrogens of the blsphosphonic acid are replaced by a pharmaceutically acceptable cation, in particular sodium, potassium or ammonium, In first instance sodium.

A very preferred group of pharmaceutically acceptable salts is characterised by having one acidic hydrogen and one pharmaceutically acceptable cation, especially sodium, in each of the phosphonic acid groups.

Zoledronic acid can also be used in the form of its hydrates or it may include other solvents used for crystallisation.

The zoledronic add is preferably administered in the form of pharmaceutical compositions that contain the 4 mg therapeutically effective amount optionally together with or in admixture with Inorganic or organic, solid or liquid, pharmaceutically acceptable carriers which are suitable for intravenous administration.

Zoledronic acid for use according to the instant invention is for the intravenous administration.

The intravenous formulations used in the instant invention are injectable fluids which are preferably isotonic aqueous solutions which can be prepared before use by methods well known in the art, for example from lyophilised preparations which contain active ingredient alone or together with a pharmaceutically acceptable carrier. The pharmaceutical preparations may be sterilised and/or contain adjuncts, for example preservatives, stabilisers, wetting agents and/or emulsifiers, solubilisers, salts for regulating the osmotic pressure and/or buffers. Preferred intravenous infusion solutions are those containing 4 mg of zoledronic add per unit dose In an infusion solution volume of from about 5 up to about 200 ml, preferably from about 50 to about 100ml and more preferably about 100 ml for infusion over a period of about 15 minutes plus or minus up to about 45 seconds.

Preferably, the compositions of the invention comprise a buffering agent. The type and amount of buffering agent may be selected in order to obtain a pH in the range of from 5 to 7, e.g., pH 5.9, when the composition of the invention is dissolved, e.g., in water. We have found that stability is best at these pH values. The pH may also be adjusted by using a basic solution, e.g., a sodium *hydroxide* solution. As a preferred buffering agent one may use trisodium citrate.

It is also preferred that the compositions of the invention comprise a bulking agent which preferably also acts as an isotonising agent. Preferably, the bulking/isotonising agent is chemically inert, has a low hygroscopicity and good bulking properties. The amount of bulking/isotonising may be selected in order to obtain an isotonic solution when the composition of the invention is dissolved. For example the weight ratio of Zoledronic acid to the buiking/isotonising agent is in the range of from 1:5 to 1:4000, e.g., 1:10 to 1:3000, e.g., 1:20 to 1:2500, e.g., 1:30 to 1:1000, e.g.,1:40 to 1:500, e.g., 1:50 to 1: 150. As a preferred bulking/isotonising agent one may use mannitol.

The intravenous solution may be obtained by dissolving a pharmaceutical composition as described above into an appropriate amount of a biocompatible water-based solvent, water or saline for parenteral administration.

For treatment of tumor induced hypercalcemia, zoledronic acid is preferably administered one time in most patients. Repeat dosing, administered no sooner than 7 days post initial treatment, normally limited to one occurrence, may be used for improved control of the hypercalcemia. For the treatment and prevention of bone metastases, long-term administration of zoledronic acid is generally administered at 3 to 4-week Intervals and at monthly intervals for patients with multiple myeloma although this can be more or less frequent depending upon individual circumstances.

The compositions of the invention may be prepared as follows. The required amount of isotonising agent is dissolved in, e.g., 50 to 90%, of the total amount of biocompatible water-based solvent or water. Zoledronic acid is then suspended and dissolved by adding a solution of the buffering agent. After complete dissolution the pH-value is adjusted to the desired range with the required amount of buffering agent. The solution is then made up to the final volume with biocompatible water-based solvent or water for parenteral use. Then the process is continued under aseptic conditions. The solution is sterilised and filled into 4 to 10 ml vials. In plastic vials, the solutions may be stored for a long-term period.

Before storage, the content of the vials may also be dried, e.g., freeze-dried, according to a pre-programmed cycle. When the cycle is complete, the vials are stoppered after flushing with a gas, e.g., nitrogen or carbon dioxide, within a chamber. The vials are sealed, e.g., with aluminium caps, outside the sterile area. The lyophilisate may then be used by re-dissolving the content of the vials into a solvent suitable for Intravenous administration, e.g. saline.

The present invention is illustrated by the following examples.

### Reference Example 1

In a stainless steel kettle the required amount of mannitol is dissolved by stirring and under nitrogen flushing in approximately 70% of the total amount of water for Injection. Zoledronic acid is suspended and dissolved by adding a 10% trisodium citrate solution. After complete dissolution the pH-value is adjusted with 10% trisodium citrate solution to 5.9 - 6.1. The solution is then made up to the final volume with water for injection. The following procedure is carried out under aseptic conditions (in a grade A dean area): The solution is passed through a sterile membrane filter (0.22 micrometers pore size), and filled into 6 ml vials with a filling weight of 1.945 g (including an overfill of 3% of the solution to be lyophilised in order to compensate for the amount of reconstituted solution remaining in the vial after withdrawal). The overfill is selected according to USP. The vials are freeze-dried according to the following cycle :

| **Step** | **Time [min.]** | **Temperature [°C]** | **Pressure [mbar]** |
|---|---|---|---|
| Begin freezing | 30 min. | -20°C | - |
| Freezing | 90 min. | -45°C | - |
| Freezing equilibration time | 150 min. | -45°C | - |
| Begin primary drying | 35 min. | -10°C | 1500 |
| Primary drying | 85 min. | 13°C | 1500 |
| Primary drying | 480 min. | 13°C | 1500 |
| Begin secondary drying | 30 min. | 30°C | 420 |
| Secondary drying | 300 min. | 30°C | 420 |
| Cooling down | 30 min. | 20°C | 420 |
| End of cycle | 5 min. | 20°C | 420 |

When the cycle is complete, the vials are stoppered after flushing with nitrogen within the chamber. The vials are sealed with aluminium caps outside the sterile area (under LF). A solution for intravenous injection may be reconstituted by adding 5 ml of sterile water for injection USP to each vial. This dose is further diluted in 50 ml of sterile 0.9% Sodium Chloride, USP, or 5% Dextrose injection, USP. If not used immediately, for microbiological integrity, the solution is refrigerated at, e.g., 36°- 46°F (2-8°C). The total time between reconstitution, dilution, storage in the refrigerator and end of administration does not preferably exceed 24 hours.

### Reference Example 2

According to a similar processing as in reference example 1 the following composition may be obtained.

| Compositions | |
|---|---|
| Zoledronic acid | 4 mg |
| Mannitol, pyrogen-free | 220.0 mg |
| Trisodium citrate | up to pH 5.9 |
| Sodium hydroxide | - |
| Water for injection | up to 1.8 ml |

The Composition of Reference Example 2 may be freeze dried and re-constituted as described in Reference Example 1.

### Example 3

4 mg zoledronic acid is reconstituted in 100 ml of infusion solution. The solution is infused over a period of 15 minutes. This corresponds to an infusion rate of drug of 4 mg / 15 minutes = 0.27 mg/minute =1 micromole/minute. The 4 mg dose, infused over 15 minutes, represents a more practical alternative to other bisphosphonate drugs, which are infused over a considerably longer period of time and at a higher infusion rate In terms of mass per unit time (mg/minute) and number of molecules per unit time (micromoles/minute), as tabulated below:

| | Bisphosphonate Drug | |
|---|---|---|
| | zoledronic acid | pamidronate |
| Clinical dose (mg) | 4 | 90 |
| Infusion time (minutes) specified in label | 15 | 120 |
| Infusion rate | | |
| mg/minute | 0.27 | 0.75 |
| micromoles/minute | 1 | 2 |

1. The volume of 100 ml containing 4 mg zoledronic acid is infused over 15 minutes as an intravenous drip, which allows the drug to be administered at a uniform and precise rate. Clinical use of shorter infusion times of zoledronic acid, for example as an intravenous push of 5 minutes [Major P, Lortholary A, Hon J,. et al., Zoledronic acid is superior to pamidronate in the treatment of hypercalcemia of malignancy: a pooled analysis of two randomized, controlled clinical trials, J Clin Oncol 2001; 19: 558-567], may result in more variable infusion rates. Changing the infusion rate impacts the peak plasma concentrations achieved, as demonstrated in a pharmacokinetic study of zoledronic acid in cancer patients with bone metastases. Relevant data are shown in the figure below.
   Plasma concentrations in patients after 4 mg zoledronic acid infused over 5 minutes and 15 minutes (mean data for n = 5 patients in 4 mg/5 min group, n = 7 patients in 4 mg/15 min group, study 503 [2]

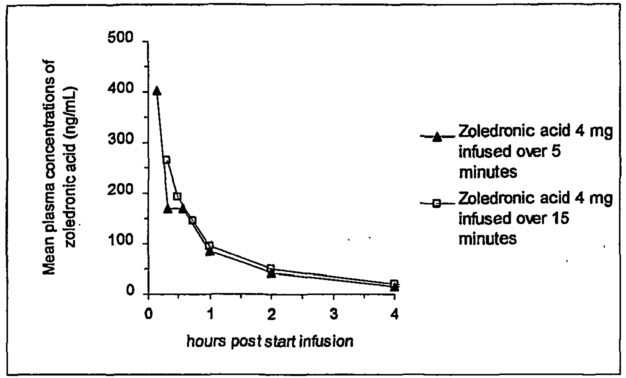

The pharmacokinetic parameters derived from the individual patients' plasma concentration versus time curves, and urinary excretion of drug are tabulated below. Differences in mean Cmax (maximum observed zoledronic acid concentration, at end of infusion) were statistically significant. Differences in AUC (area under zoledronic acid concentration versus time curve) and Ae (amount of drug excreted in urine) were not significant.

| | Zoledronic acid 4 mg infused over 15 minutes (n=7) | Zoledronic acid 4 mg infused over 5 minutes (n=5) |
|---|---|---|
| Cmax (ng/ml) | 264 ± 86 | 403 ± 118 |
| AUC 0-24 h (ngxh/ml) | 420 ± 218 | 378 ± 116 |
| Ae 0-24 h (% of dose) | 37.4 ± 17.0 | 38.7 ± 6.34 |

The 4 mg dose of zoledronic acid has been determined to be clinically effective in several controlled trials in cancer patients, with placebo or the bisphosphonate pamidronate as comparator. Pertinent data attesting to zoledronic acid showing comparable efficacy to pamidronate and superior efficacy against placebo are summarized below.

| | Percentage of patients showing a skeletal related adverse event | | Statistical significance |
|---|---|---|---|
| | Zoledronic acid 4 mg | Comparator: Placebo or pamidronate | |
| Study 10: | | | |
| breast cancer patients | 42% | 47% | P>0.05 |
| multiple myeloma patients | 47% | 49% | P>0.05 |

| Study 11: | | | |
|---|---|---|---|
| hypercalcemic lung cancer patients | 42% | 48% | P=0.036 |
| hypercalcemic patients with other solid tumors | 33% | 46% | P = 0.047 |

| Study 39: | | | |
|---|---|---|---|
| prostate cancer patients | 33% | 44% | P = 0.021 |

The dose of 4 mg zoledronic acid infused over 15 minutes offers a safety advantage in terms of renal tolerability over the shorter infusion time of 5 minutes. The lower incidence of renal adverse events with zoledronic acid infused over 15 minutes versus 5 minutes is summarized below. Renal adverse events are defined as increases in a patient's serum creatinine over baseline, by ≥0.5 mg/dL if baseline <1.4 mg/dL, by ≥1 mg/dL if baseline ≥ 1.4 mg/dL, by ≥ 2-fold irrespective of baseline value.

| | zoledronic acid 4 mg | |
|---|---|---|
| | 15 minutes infusion | 5 minutes infusion |
| Study 10: | 24/272 patients | 36/272 patients |
| breast cancer and multiple myeloma patients | 8.8% | 13.2% |

| Study 11: | 18/165 patients | 10/61 patients |
|---|---|---|
| lung cancer patients | 10.9% | 16.4% |

| Study 39: | 14/78 patients | 22/111 patients |
|---|---|---|
| prostate cancer patients | 15.2% | 19.8% |

Zoledronic acid 4 mg infused over 15 minutes showed similar renal tolerability compared to pamidronate 90 mg infused over 2 hours, 8.8% versus 8.2%, respectively.

4 mg of zoledronic acid infused over 15 minutes versus higher doses of zoledronic acid, 8 mg and 16 mg results in improved renal safety without, surprisingly, losing clinical efficacy.

In summary, 4 mg zoledronic acid infused in a 100 mL volume over 15 minutes offers an input rate of drug into the patients' systemic circulation of 1 micromole per minute, which can be precisely administered and is considerably lower than the infusion rate used for other bisphosphonate drugs. The shorter duration of the infusion, 15 minutes for zoledronic acid versus 2 hours for pamidronate, offers a greater degree of flexibility and practicality in the clinical setting. The clinical studies attest to a clinical benefit in the choice of zoledronic acid 4 mg dose infused over 15 minutes in terms of improved renal tolerability versus a shorter infusion period of 5 minutes, but without impacting clinical efficacy, which is comparable to the current standard therapy pamidronate, and superior to placebo.

## Claims

1. 2-(imidazol-1yl)-1-hydroxyethane-1,1-diphosphonic acid or pharmaceutically acceptable salts thereof for use in the treatment of bone metabolism disease, wherein 4 mg of 2-(imidazol-1yl)-1-hydroxyethane-1,1-diphosphonic acid is to be administered intravenously over a period of 15 minutes in an infusion volume of 100 ml to a patient in need of said treatment.

2. 2-(imidazol-1 yl)-1-hydroxyethane-1,1-diphosphonic acid or pharmaceutically acceptable salts thereof for use in the treatment of bone metabolism disease according to claim 1, wherein the bone metabolism disease is tumour induced hypercalcemia, prosthesis loosening or tumour angiogenesis.

3. 2-(imidazol-1yl)-1-hydroxyethane-1,1-diphosphonic acid or pharmaceutically acceptable salts thereof for use in the treatment of bone metabolism disease according to claim 2, wherein the bone metabolism disease is tumour induced hypercalcemia.

4. 2-(imidazol-1yl)-1-hydroxyethane-1,1-diphosphonic acid or pharmaceutically acceptable salts thereof for use in the treatment of bone metastases or multiple myeloma, wherein 4 mg of 2-(imidazol-1yl)-1-hydroxyethane-1,1-diphosphonic acid is to be administered intravenously over a period of 15 minutes in an infusion volume of 100 ml to a patient in need of said treatment.

5. A pharmaceutical composition containing 4 mg of therapeutically effective 2-(imidazol-1yl)-1-hydroxyethane-1,1-diphosphonic acid for use in the treatment of bone metabolism disease, wherein 4 mg of 2-(imidazol-1yl)-1-hydroxyethane-1,1-diphosphonic acid is to be administered intravenously over a period of 15 minutes in an infusion volume of 100 ml to a patient in need of said treatment.

6. A pharmaceutical composition containing 4 mg of therapeutically effective 2-(imidazol-1 yl)-1-hydroxyethane-1,1-diphosphonic acid for use in the treatment of bone metabolism disease according to claim 5, wherein bone metabolism disease is tumour induced hypercalcemia, prosthesis loosening or tumour angiogenesis.

7. A pharmaceutical composition containing 4 mg of therapeutically effective 2-(imidazol-1yl)-1-hydroxyethane-1,1-diphosphonic acid for use in the treatment of bone metabolism disease according to claim 6, wherein the bone metabolism disease is tumour induced hypercalcemia.

8. A pharmaceutical composition containing 4 mg of therapeutically effective 2-(imidazol-1 yl)-1-hydroxyethane-1,1-diphosphonic acid for use in the treatment of bone metastases or multiple myeloma, wherein 4 mg of 2-(imidazol-1yl)-1-hydroxyethane-1,1-diphosphonic acid is to be administered intravenously over a period of 15 minutes in an infusion volume of 100 ml to a patient in need of said treatment.

## Patentansprüche

1. 2-(Imidazol-1-yl)-1-hydroxyethan-1,1-diphosphansäure oder pharmazeutisch akzeptable Salze hiervon zur Verwendung bei der Behandlung einer Knochenmetabolismuserkrankung, wobei 4 mg 2-(Imidazol-1-yl)-1-hydroxyethan-1,1-diphosphonsäure intravenös über einen Zeitraum von 15 min in einem Infusionsvolumen von 100 ml an einen Patienten mit Bedarf für diese Behandlung zu verabreichen sind.

2. 2-(Imidazol-1-yl)-1-hydroxyethan-1,1-diphosphonsäure oder pharmazeutisch akzeptable Salze hiervon zur Verwendung bei der Behandlung einer Knochenmetabolismuserkrankung nach Anspruch 1, wobei die Knochenmetabolismuserkrankung eine Tumor-induzierte Hyperkalziämie, Prothesenlockerung oder Tumor-Angiogenese ist.

3. 2-(Imidazol-1-yl)-1 -hydroxyethan-1,1-diphosphonsäure oder pharmazeutisch akzeptable Salze hiervon zur Verwendung bei der Behandlung einer Knochenmetabolismuserkrankung nach Anspruch 2, wobei die Knochenmetabolismuserkrankung eine Tumor-induzierte Hyperkalziämie ist.

4. 2-(Imidazol-1-yl)-1-hydroxyethan-1,1-diphosphonsäure oder pharmazeutisch akzeptable Salze hiervon zur Verwendung bei der Behandlung von Knochenmetastasen oder multiplem Myelom, wobei 4 mg 2-(Imidazol-1-yl)-1-hydroxyethan-1,1-diphosphonsäure intravenös über einen Zeitraum von 15 min in einem Infusionsvolumen von 100 ml an einen Patienten mit Bedarf für diese Behandlung zu verabreichen sind.

5. Pharmazeutische Zusammensetzung, die 4 mg einer therapeutisch wirksamen 2-(Imidazol-1-yl)-1-hydroxyethan-1,1-diphosphonsäure enthält, zur Verwendung bei der Behandlung einer Knochenmetabolismuserkrankung, wobei 4 mg 2-(Imidazol-1-yl)-1-hydroxyethan-1,1-diphosphonsäure intravenös über einen Zeitraum von 15 min in einem Infusionsvolumen von 100 ml an einen Patienten mit Bedarf für diese Behandlung zu verabreichen sind.

6. Pharmazeutische Zusammensetzung, die 4 mg einer therapeutisch wirksamen 2-(Imidazol-1-yl)-1-hydroxyethan-1,1-diphosphonsäure enthält, zur Verwendung bei der Behandlung einer Knochenmetabolismuserkrankung nach Anspruch 5, wobei die Knochenmetabolismuserkrankung eine Tumor-induzierte Hyperkalziämie, Prothesenlockerung oder Tumor-Angiogenese ist.

7. Pharmazeutische Zusammensetzung, die 4 mg einer therapeutisch wirksamen 2-(Imidazol-1-yl)-1-hydroxyethan-1,1-diphosphonsäure enthält, zur Verwendung bei der Behandlung einer Knochenmetabolismuserkrankung nach Anspruch 6, wobei die Knochenmetabolismuserkrankung eine Tumor-induzierte Hyperkalziämie ist.

8. Pharmazeutische Zusammensetzung, die 4 mg einer therapeutisch wirksamen 2-(Imidazol-1-yl)-1-hydroxyethan-1,1-diphosphonsäure enthält, zur Verwendung bei der Behandlung von Knochenmetastasen oder multiplem Myelom, wobei 4 mg 2-(Imidazol-1 -yl)-1 - hydroxyethan-1,1-diphosphonsäure intravenös über einen Zeitraum von 15 min in einem Infusionsvolumen von 100 ml an einen Patienten mit Bedarf für diese Behandlung zu verabreichen sind.

## Revendications

1. Acide 2-(imidazol-1-yl)-1-hydroxyéthane-1,1-diphosphonique ou sels de celui-ci acceptables sur le plan pharmaceutique, à utiliser dans le traitement d'une maladie du métabolisme osseux, où 4 mg d'acide 2-(imidazol-1-yl)-1-hydroxyémane-1,1-diphosphonique doivent être administrés à un patient ayant besoin d'un tel traitement, par voie intraveineuse, sur une période de 15 minutes, dans un volume de perfusion de 100 ml.

2. Acide 2-(imidazol-1-yl)-1-hydroxyéthane-1,1-diphosphonique ou sels de celui-ci acceptables sur le plan pharmaceutique, à utiliser dans le traitement d'une maladie du métabolisme osseux selon la revendication 1, où la maladie du métabolisme osseux est une hypercalcémie induite par des tumeurs, un descellement de prothèse ou une angiogenèse tumorale.

3. Acide 2-(imidazol-1-yl)-1-hydroxyéthane-1,1-diphosphonique ou sels de celui-ci acceptables sur le plan pharmaceutique, à utiliser dans le traitement d'une maladie du métabolisme osseux selon la revendication 2, où la maladie du métabolisme osseux est une hypercalcémie induite par des tumeurs.

4. Acide 2-(imidazol-1-yl)-1-hydroxyéthane-1,1-diphosphonique ou sels de celui-ci acceptables sur le plan pharmaceutique, à utiliser dans le traitement de métastases osseuses ou d'un myélome multiple, où 4 mg d'acide 2-(imidazol-1-yl)-1-hydroxyéthane-1,1-diphosphonique doivent être administrés à un patient ayant besoin d'un tel traitement, par voie intraveineuse, sur une période de 15 minutes, dans un volume de perfusion de 100 ml.

5. Composition pharmaceutique contenant 4 mg d'acide 2-(imidazol-1-yl)-1-hydroxyéthane-1,1-diphosphonique efficace sur le plan thérapeutique, à utiliser dans le traitement d'une maladie du métabolisme osseux, où 4 mg d'acide 2-(imidazol-1-yl)-1-hydroxyéthane-1,1-diphosphonique doivent être administrés à un patient ayant besoin d'un tel traitement, par voie intraveineuse, sur une période de 15 minutes, dans un volume de perfusion de 100 ml.

6. Composition pharmaceutique contenant 4 mg d'acide 2-(imidazol-1-yl)-1-hydroxyéthane-1,1-diphosphonique efficace sur le plan thérapeutique, à utiliser dans le traitement d'une maladie du métabolisme osseux selon la revendication 5, où la maladie du métabolisme osseux est une hypercalcémie induite par des tumeurs, un descellement de prothèse ou une angiogenèse tumorale.

7. Composition pharmaceutique contenant 4 mg d'acide 2-(imidazol-1-yl)-1-hydroxyéthane-1,1-diphosphonique efficace sur le plan thérapeutique, à utiliser dans le traitement d'une maladie du métabolisme osseux selon la revendication 6, où la maladie du métabolisme osseux est une hypercalcémie induite par des tumeurs.

8. Composition pharmaceutique contenant 4 mg d'acide 2-(imidazol-1-yl)-1-hydroxyéihane-1,1-diphosphonique efficace sur le plan thérapeutique, à utiliser dans le traitement de métastases osseuses ou d'un myélome multiple, où 4 mg d'acide 2-(imidazol-1-yl)-1-hydroxyéthane-1,1-diphosphonique doivent être administrés à un patient ayant besoin d'un tel traitement, par voie intraveineuse, sur une période de 15 minutes, dans un volume de perfusion de 100 ml.
